(19) Europäisches Patentamt / European Patent Office / Office européen des brevets

(11) **EP 4 142 812 B1**

(12) **EUROPEAN PATENT SPECIFICATION**

(45) Date of publication and mention
of the grant of the patent:
**22.05.2024   Bulletin 2024/21**

(21) Application number: **21721476.6**

(22) Date of filing: **22.04.2021**

(51) International Patent Classification (IPC):
***A61L 2/14*** (2006.01)

(52) Cooperative Patent Classification (CPC):
**H05H 1/463; A61L 2/14; H05H 1/466;**
A61L 2202/11; A61L 2202/16; A61L 2202/24;
A61L 2202/25; H05H 2245/34; Y02A 50/30

(86) International application number:
**PCT/EP2021/060502**

(87) International publication number:
**WO 2021/219482 (04.11.2021 Gazette 2021/44)**

(54) **STERILISATION APPARATUS FOR PRODUCING PLASMA AND HYDROXYL RADICALS**

STERILISATIONSVORRICHTUNG ZUR ERZEUGUNG VON PLASMA- UND
HYDROXYLRADIKALEN

APPAREIL DE STÉRILISATION POUR LA PRODUCTION DE PLASMA ET DE RADICAUX
HYDROXYLES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **30.04.2020   GB 202006384**

(43) Date of publication of application:
**08.03.2023   Bulletin 2023/10**

(73) Proprietor: **Creo Medical Limited
Chepstow, Monmouthshire NP16 5UH (GB)**

(72) Inventors:
 • **HANCOCK, Christopher
   Chepstow Monmouthshire
   South Wales NP16 5UH (GB)**
 • **TURNER, Louis
   Chepstow Monmouthshire
   South Wales NP16 5UH (GB)**

 • **PRESTON, Shaun
   Chepstow Monmouthshire
   South Wales NP16 5UH (GB)**
 • **MEADOWCROFT, Simon
   Chepstow Monmouthshire
   South Wales NP16 5UH (GB)**
 • **ULLRICH, George Christian
   Gwynedd, Wales LL57 4YY (GB)**
 • **WEBB, David Edward
   Gwynedd, Wales LL57 4YY (GB)**

(74) Representative: **Mewburn Ellis LLP
Aurora Building
Counterslip
Bristol BS1 6BX (GB)**

(56) References cited:
**WO-A1-2019/175063      GB-A- 2 463 521
GB-A- 2 548 382**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

FIELD OF THE INVENTION

The invention relates to sterilisation systems suitable for clinical use, e.g. on the human body, medical apparatuses, or hospital bed spaces. For example, the invention may provide a system that can be used to destroy or treat certain bacteria and/or viruses associated with the human or animal biological system and/or the surrounding environment. This invention is particularly useful for sterilising or decontaminating enclosed or partially enclosed spaces.

BACKGROUND TO THE INVENTION

Bacteria are single-celled organisms that are found almost everywhere, exist in large numbers and are capable of dividing and multiplying rapidly. Most bacteria are harmless, but there are three harmful groups; namely: cocci, spirilla, and bacilla. The cocci bacteria are round cells, the spirilla bacteria are coil-shaped cells, and the bacilli bacteria are rod-shaped. The harmful bacteria cause diseases such as tetanus and typhoid.

Viruses can only live and multiply by taking over other cells, i.e. they cannot survive on their own. Viruses cause diseases such as colds, flu, mumps and AIDS. Viruses may be transferred through person-to-person contact, or through contact with region that is contaminated with respiratory droplets or other virus-carrying bodily fluids from an infected person.

Fungal spores and tiny organisms called protozoa can cause illness.

Sterilisation is an act or process that destroys or eliminates all form of life, especially micro-organisms. During the process of plasma sterilisation, active agents are produced. These active agents are high intensity ultraviolet photons and free radicals, which are atoms or assemblies of atoms with chemically unpaired electrons. An attractive feature of plasma sterilisation is that it is possible to achieve sterilisation at relatively low temperatures, such as body temperature. Plasma sterilisation also has the benefit that it is safe to the operator and the patient.

Plasma typically contains charged electrons and ions as well as chemically active species, such as ozone, nitrous oxides, and hydroxyl radicals. Hydroxyl radicals are far more effective at oxidizing pollutants in the air than ozone and are several times more germicidal and fungicidal than chlorine, which makes them a very interesting candidate for destroying bacteria or viruses and for performing effective decontamination of objects contained within enclosed spaces, e.g. objects or items associated with a hospital environment.

OH radicals held within a "macromolecule" of water (e.g. a droplet within a mist or fog) are stable for several seconds and they are 1000 times more effective than conventional disinfectants at comparable concentrations.

An article by Bai et al titled "Experimental studies on elimination of microbial contamination by hydroxyl radicals produced by strong ionisation discharge" (Plasma Science and Technology, vol. 10, no. 4, August 2008) considers the use of OH radicals produced by strong ionisation discharges to eliminate microbial contamination. In this study, the sterilisation effect on E. coli and B. subtilis is considered. The bacteria suspension with a concentration of $10^7$ cfu/ml (cfu = colony forming unit) was prepared and a micropipette was used to transfer 10 $\mu$l of the bacteria in fluid form onto 12 mm $\times$ 12 mm sterile stainless steel plates . The bacteria fluid was spread evenly on the plates and allowed to dry for 90 minutes. The plates were then put into a sterile glass dish and OH radicals with a constant concentration were sprayed onto the plates. The outcomes from this experimental study were:

1. OH radicals can be used to cause irreversible damage to cells and ultimately kill them;
2. The threshold potential for eliminating micro-organisms is ten thousandths of the disinfectants used at home or abroad;
3. The biochemical reaction with OH is a free radical reaction and the biochemical reaction time for eliminating micro-organisms is about 1 second, which meets the need for rapid elimination of microbial contamination, and the lethal time is about one thousandth of that for current domestic and international disinfectants;
4. The lethal density of OH is about one thousandths of the spray density for other disinfectants - this will be helpful for eliminating microbial contamination efficiently and rapidly in large spaces, e.g. bed-space areas; and
5. The OH mist or fog drops oxidize the bacteria into $CO_2$, $H_2O$ and micro-inorganic salts. The remaining OH will also decompose into $H_2O$ and $O_2$, thus this method will eliminate microbial contamination without pollution.

WO 2009/060214 discloses sterilisation apparatus arranged controllably to generate and emit hydroxyl radicals. The apparatus includes an applicator which receives RF or microwave energy, gas and water mist in a hydroxyl radical generating region. The impedance at the hydroxyl radical generating region is controlled to be high to promote creation of an ionisation discharge which in turn generates hydroxyl radicals when water mist is present. The applicator may be a coaxial assembly or waveguide. A dynamic tuning mechanism e.g. integrated in the applicator may control the impedance at the hydroxyl radical generating region. The delivery means for the mist, gas and/or energy can be integrated with each other.

WO 2019/175063 discloses a sterilisation apparatus that uses thermal or non-thermal plasma to sterilise or disinfect surgical scoping devices. In one example, a plasma generating region is formed at a distal end of a coaxial transmission line, which convey RF or microwave energy to strike and sustain the plasma. A gas passageway is formed around an outer surface of the coaxial transmission line. The gas passageway is in fluid communication with the plasma generating region through notches in a cylindrical electrode mounted on a distal end of the coaxial transmission line. In some examples, water through a passageway formed within the inner conductor of the coaxial transmission line, from where it is sprayed on to the surface of an object before the plasma passes over it.

WO 2019/175063 A1 describes a sterilisation apparatus which uses thermal or non-thermal plasma to disinfect surgical scoping devices such as endoscopes, gastroscopes, laparoscopes and the like.

GB 2548382 A discloses a plasma torch having an open end comprising a tapered central cathode rod and a grounded conductive tube, the grounded conductive tube has an open end and is arranged around the cathode and spaced therefrom to form a first cylindrical cavity open at one end; in use, an arc discharge between the cathode and grounded conductive tube ionizes a feed gas to produce a central thermal plasma emitted from the open end of the first cavity.

GB 2463521 A relates to a system and method for producing concentrations of OH radicals to decontaminate sections of hospital wards, hospital bed-spaces or operating theatres.

## SUMMARY OF THE INVENTION

At its most general, the invention provides a sterilisation device that supports an inline fluid feed that is used to generate a thermal or non-thermal plasma in the presence of water to provide a stream containing hydroxyl radicals. The stream may be directed on to a surface or object to perform sterilisation. By providing the fluid feed (which may comprise a gas feed and/or a water feed) to the device inline with the direction the stream is output, a stronger flow of hydroxyl radicals and hence a greater range of sterilisation may be achieved.

According to a first aspect of the invention, there is provided sterilisation device for generating a flow of hydroxyl radicals, the sterilisation device comprising: a coaxial transmission line for conveying radiofrequency (RF) and/or microwave frequency electromagnetic (EM) energy, the coaxial transmission line extending in a longitudinal direction and comprising an inner conductor and an outer conductor located around and spaced away from the inner conductor; an end cap mounted on a distal end of the coaxial transmission line, wherein the end cap comprises a distally facing outlet aperture; a fluid conduit extending in the longitudinal direction from a fluid inlet at a distal end of the coaxial transmission line through the end cap to the outlet aperture; and a plasma generating region at a proximal end of the outlet aperture, wherein the plasma generating region contains a first electrode that is electrically connected to the inner conductor, and a second electrode that is electrically connected to the outer conductor, wherein the fluid conduit defines a longitudinal fluid flow path through the device that is aligned with a feed direction in which fluid is receivable through the fluid inlet, and wherein the first electrode and second electrode oppose each other in a transverse direction across the longitudinal fluid flow path in the plasma generating region. With this arrangement, the fluid flow may be substantially uninterrupted through the device.

The fluid conduit may be arranged to supply a mixture of an inert gas and a water mist to the plasma generating region. Alternatively, in some embodiments or operating modes of the device as described below, the inert gas and water may be supplied separately to the plasma generating region. For example the fluid conduit may be arranged to supply only an inert gas to the plasma generating region.

The device may be configured to generate and emit hydroxyl radicals. The device is preferably configured as a handheld unit, so that it can be easily used to sterilise any surface or object as required. In particular, gas which is supplied to the plasma generating region may be used to generate a thermal or non-thermal plasma using RF and/or microwave frequency. For example, the inner conductor and the outer conductor are arranged to produce a high electric field from the received RF and/or microwave frequency energy across the flow path for the gas to strike and sustain a thermal or non-thermal plasma. For example, a short pulse (e.g. having a duration of 10 ms or less, e.g. between 1 ms and 10 ms) of RF energy may be used to strike the plasma. A longer microwave pulse may be used to sustain the plasma. It may also be possible to strike the plasma using the microwave frequency energy, e.g. by using a microwave resonator or an impedance transformer, i.e. a quarter wave transformer that transforms a low voltage to a higher voltage to strike plasma using a higher impedance transmission line that is a quarter wave (or an odd multiple thereof) long at the frequency of operation. This high impedance line may be switched in to strike plasma and switched out (i.e. to return to a lower impedance line) once the plasma has been struck and it is required to sustain plasma. A power PIN or varactor diode may be preferably used to switch between the two states, although it may be possible to use a co-axial or waveguide switch. The thermal or non-thermal plasma which is produced can ionise water which is also delivered to the plasma generating region (for example, in combination with the inert gas or separately) in order to produce hydroxyl radicals. The hydroxyl radicals are directed out of the outlet aperture in order to sterilise surfaces or objects as required by a user.

The fluid conduit may include a passage between the inner conductor and outer conductor of the coaxial transmission line. This arrangement may be beneficial for forming a compact device. An inert gas conveyed by the fluid conduit may thus form a dielectric material for the coaxial transmission line. In this arrangement, the inner conductor may be separated from the outer conductor by a plurality of radial spokes mounted within the coaxial transmission line, e.g. at intervals of a half wavelength of the microwave energy conveyed thereby.

In another embodiment, the fluid conduit may include a duct that runs parallel to the coaxial transmission line. In other words, the gas may be supplied independently of the coaxial transmission line.

The device may include a water conduit, e.g. separately to the fluid conduit, arranged to deliver water to the plasma generating region. The water conduit may include a longitudinal passageway formed within the inner conductor of the coaxial transmission line. That is, the inner conductor of the coaxial feedline may be hollow to define a water conduit for conveying water to the plasma generating region. The fluid conduit may thus be arranged to convey gas to the plasma generating region. In one example, the water may be supplied through the inner conductor as a water mist, which may aid the generation of hydroxyl radicals as water droplets are more readily ionised and dispersed.

The water conduit may further include a longitudinal passageway formed within the first electrode. The device may comprise a spray unit at a distal end of the water conduit. The spray unit may comprise an aerosoliser configured to produce a cone-shaped spray of water mist. Water may be conveyed through the water conduit as a stream of water to be turned into a water mist at the distal end by the aerosoliser. The water mist may be sprayed directly into the plasma generating region. Preferably, the aerosoliser may be configured to produce a cone-shaped mist to ensure water droplets are dispersed in a large volume of the plasma generating region. For example, the aerosoliser may comprise a number (e.g. one or more, preferably two) striking surfaces in the path of the water stream to induce rotational motion, thereby generating a vortex of water which breaks the water stream into a mist of water droplets due to the water stream striking the surface/s and also due to centrifugal forces in the vortex. Conveying a stream of water through the inner conductor in this way may allow water to be delivered at a high pressure, increasing the rate at which hydroxyl radicals can be generated and also aiding the dispersal of radicals over a larger area as they leave the device through the outlet aperture.

The water conduit may have a proximal inlet, wherein the water conduit defines a longitudinal flow path through the device that is aligned with a feed direction in which water is receivable through the proximal inlet. That is, water may be fed to the water conduit parallel to the longitudinal axis of the inner conductor, which may allow water to be delivered to the device at a higher pressure (e.g. in comparison with a water feed connected to the inner conductor at an angle). For the same reason, it is particularly preferable for the water conduit to be substantially straight along its length.

The first electrode may be an electrically conductive rod that protrudes in the longitudinal direction from a distal end of the inner conductor, the rod having a smaller diameter than the inner conductor. Where the first electrode conveys water, the speed of water through the water conduit may be accelerated through the rod before reaching the spray unit, further aiding dispersion of the water mist and of the hydroxyl radicals.

The plasma generating region may be located in a proximal region of the outlet aperture. The end cap may comprise a generally cylindrical conductive element having the outlet aperture in a distal end surface thereof. The end cap may define an internal volume into which the inner conductor of the coaxial transmission line protrudes beyond a distal end of the outer conductor. The end cap may be electrically connected to the outer conductor. A diameter of the outlet aperture may be less than a diameter of the internal volume.

The second electrode may be integrally formed with the end cap. In one example the second electrode comprises a plurality of radial tabs extending inwards from a side wall of the outlet aperture.

The sterilisation device may further comprise an insulating tube mounted in the outlet aperture distally from the plasma generating region, e.g. beyond the second electrode. The insulating tube (e.g. made from quartz or the like) may prevent unwanted plasma strikes in the outlet aperture beyond the plasma generating region.

In order to make space for the inline fluid feed, the RF and/or microwave energy may be supplied to the coaxial transmission line via one or more transverse feeds.

For example, the device may comprise a transverse coaxial feed connected to introduce the RF and/or microwave energy to the coaxial transmission line in a proximal region thereof. The transverse coaxial feed may be configured to couple microwave energy into the coaxial transmission line, and wherein the transverse coaxial feed is connected to a point on the coaxial transmission line located at a distance $\frac{(2n-1)\lambda}{4}$ from a proximal end thereof, where n is a positive integer, and $\lambda$ is a wavelength of the microwave energy conveyed by the coaxial transmission line.

The device may further comprise a proximal transverse coaxial feed connected to introduce the RF energy directly to the plasma generating region, e.g. to facilitate a plasma strike. The proximal transverse coaxial feed may comprise a fine electrode which protrudes into a space between the first electrode and the second electrode in the plasma generating region.

The device may comprise a choke mounted at a proximal end of the coaxial transmission line.

In another aspect, there may be provided a sterilisation apparatus comprising: the sterilisation device described above; a water supply connected to supply water to the plasma generating region; a gas supply connected to supply gas to the plasma generating region via the fluid conduit; and a generator connected to supply radiofrequency (RF) and/or microwave frequency electromagnetic energy to the plasma generating region. RF EM energy may be for striking the plasma, and may be received as a high voltage pulse. The microwave EM energy may be for sustaining the plasma, i.e. delivering power into the plasma to maintain the state of ionisation. This may also be received as a pulse. The plasma may be struck repeatedly in a manner to produce a quasi-continuous beam of plasma. As water is provided to the sterilisation device, the apparatus does not need to use any chemical cleaning agents, and so no harmful by-products result from sterilisation using the present apparatus.

In certain embodiments, the water supply may comprise a pump in order to supply a stream of water at a high pressure. This may be particularly preferable where the handheld sterilisation device comprises a water conduit through the inner conductor of the coaxial feedline.

In other embodiments, the water supply may comprise a mist generator. For example, the mist generator may comprise either an ultrasonic transducer or a heating element. In this way, the mist generator may supply a mist (e.g. moisture or fog) to the handheld sterilisation device for the production of hydroxyl radicals from water. Such an arrangement may be particularly preferably in arrangements where a mixture of gas and water is to be conveyed through the fluid conduit formed within the outer conductor of the coaxial feedline. Preferably, the gas supply may be connected to supply gas to the handheld sterilisation device via the mist generator. In this way, the gas supply may pressurise the flow of mist through the handheld sterilisation device to ensure a high rate of production of hydroxyl radicals and aid dispersion of the radicals from the device.

The flow rate of gas may be in the range 1.5 to 15 litres/minute, preferably between 2 and 6 litres/minute. The water supply may be arranged to generate sufficient spray or mist to form at least 2% by volume of the combined gas/water stream. The flow rate of the gas may be controlled to reach a desired proportion of gas and water in the combined stream.

Preferably the gas supply is a supply of argon gas. However, any other suitable gas may be chosen, e.g. carbon dioxide, helium, nitrogen, a mixture of air and any one of these gases, for example 10% air/90% helium.

Advantageously, the generator may be powered by a battery, such that the generator is portable. Preferably the water supply and the gas supply are also portable such that a user may easily operate the sterilisation apparatus, and sterilisation can be easily performed in any necessary environment.

Herein, the term "inner" means radially closer to the centre (e.g. axis) of the coaxial cable, probe tip, and/or applicator. The term "outer" means radially further from the centre (axis) of the coaxial cable, probe tip, and/or applicator.

The term "conductive" is used here to mean electrically conductive, unless the context dictates otherwise.

Herein, the terms "proximal" and "distal" refers to the ends of the applicator. In use, the proximal end is closer to a generator for providing the RF and/or microwave energy, whereas the distal end is further from the generator.

In this specification "microwave" may be used broadly to indicated a frequency range of 400 MHz to 100 GHz, but preferably in the range 1 GHz to 60 GHz. Specific frequencies that have been considered are: 915 MHz, 2.45 GHz, 3.3 GHz, 5.8 GHz, 10 GHz, 14.5 GHz, and 25 GHz. In contrast, this specification uses "radiofrequency" or "RF" to indicate a frequency range that is at least three orders of magnitude lower, e.g. up to 300 MHz, preferably 10 kHz to 1MHz, and most preferably 400 kHz. The microwave frequency may be adjusted to enable the microwave energy delivered to be optimised. For example, a probe tip may be designed to operate at a certain frequency (e.g. 900 MHz), but in use the most efficient frequency may be different (e.g. 866 MHz).

BRIEF DESCRIPTION OF THE DRAWINGS

Features of the invention are now explained in the detailed description of examples of the invention given below with reference to the accompanying drawings, in which:

Fig. 1 is a schematic diagram of a sterilisation apparatus according to an embodiment of the present invention;
Fig. 2 is a cross-sectional view of an applicator according to an embodiment of the present invention; and
Fig. 3 is a cross-sectional view of an applicator according to another embodiment of the present invention.

DETAILED DESCRIPTION; FURTHER OPTIONS AND PREFERENCES

This invention relates to a device for performing sterilisation using hydroxyl radicals that are generated by creating a plasma in the presence of water mist.

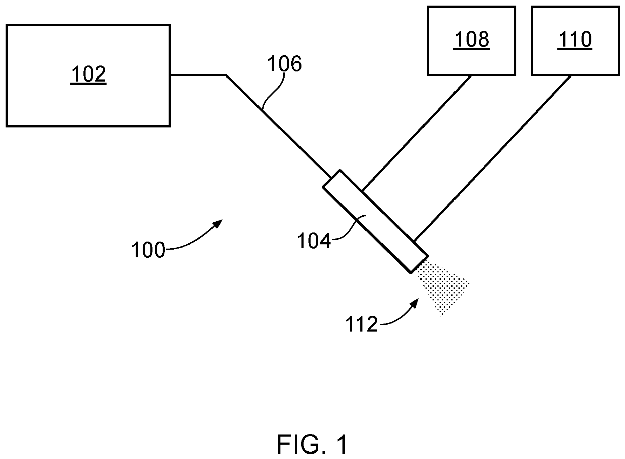

Fig. 1 is a schematic diagram of a sterilisation apparatus 100 which is an embodiment of the present invention. The apparatus 100 is capable of generating hydroxyl (OH) radicals in order to sterilise a surface or an area. For example, the apparatus 100 may be used to sterilise medical apparatuses or hospital bed spaces.

The apparatus 100 comprises a generator 102 which is able to controllably deliver radiofrequency (RF) and/or microwave electromagnetic (EM) energy to a sterilisation device, referred to herein as an applicator 104, which is preferably a handheld unit.

The generator 102 may be of the type disclosed in WO 2012/076844, for example. The generator 102 is connected to the applicator 104 by a coaxial cable 106. The coaxial cable 106 comprises an inner conductor, an outer conductor and a dielectric material separating the inner conductor from the outer conductor. The coaxial cable 106 may couple energy into the applicator 104 through a QMA connector or the like. In some examples, the generator 102 may be arranged to monitor reflected signals (i.e. reflected power) received back from the applicator 104 in order to determine an appropriate signal to be conveyed to the applicator 104. The radiofrequency and/or microwave energy is utilised at the applicator 104 in order to strike and sustain a thermal or non-thermal plasma in order to generate hydroxyl radicals in a manner which is explained in more detail below.

In some examples, the thermal or non-thermal plasma may be emitted from the applicator and usable directly to sterilise surfaces. In the apparatus shown in Fig. 1, a single generator 102 is arranged to supply RF and/or microwave frequency EM energy. However, in some embodiments of the present invention, the apparatus may comprise an RF EM energy generator and a microwave energy EM generator as individual components, which are each connected to the applicator 104 by a respective coaxial cable.

The apparatus 100 further comprises a water supply 108, which is arranged to deliver water to the applicator 104. In one example, the water may be supplied as a stream of water which may be arranged to form a spray (e.g. a shower of fine water droplets) to be emitted from the applicator 104. In another example, the water may be supplied as a water mist (e.g. moisture of fog). The water supply 108 may thus comprise a mist generator. A mist generator may generate a mist by means of an ultrasonic transducer, for example. Alternatively, the mist generator may be arranged to heat water to generate steam or mist to be passed to the applicator 104. A mist generator may include a pump or other fluid driving unit to cause generated mist to flow towards the applicator 104. The water is supplied to the applicator 104 in order to generate hydroxyl radicals by a process which will be explained in more detail below. By using water in this way the apparatus 100 can be used to sterilise surfaces or objects without the use of any cleaning chemicals, reducing costs associated with sterilisation and allowing sterilisation to be performed when cleaning chemicals are in short supply. The use of hydroxyl radicals for sterilisation also ensures that there are no harmful by-products.

A gas supply 110 is connected to the applicator 104 to supply gas for forming a plasma which is used to generate hydroxyl radicals in a manner which will be explained below. The gas supply 110 may be a pressurised supply of any suitably inert gas for formation of a non-thermal or thermal plasma, for example argon, helium, nitrogen, carbon dioxide or a combination thereof. The gas supply 110 may be configured to allow adjustment of the flow rate of gas which is delivered to the applicator 104. The gas supply can supply between 1.5 and 15 litres of gas per minute, for example.

The gas supply 110 and water supply 108 may be connected to the applicator 104 by a common feed line. That is, outputs from the gas supply 110 and water supply 108 may be combined before they reach the applicator 104. This arrangement may be particularly suitable in examples where the water supply 108 comprises a mist generator. The flow of gas from the gas supply 110 may entrain the water mist from the water supply 108 to create a combined mist/gas stream that is supplied to the applicator 104. The combined mist/gas stream may be delivered into the applicator 104 through a single fluid conduit. Alternatively, the gas supply 110 and the water supply 108 may provide separate streams for delivery of water and gas. The separate streams may be provided within a combined conduit. For example, a conduit for conveying gas to the applicator 104 may comprise a T-junction to allow water to be fed into the conduit. Alternatively, as shown in Fig. 1, the gas supply 110 and the water supply 108 are separately connected to the applicator 104.

In some embodiments of the invention it is envisaged that the generator 102 (or multiple generators where present), the mist generator 108 and the gas supply 110 may each be portable, and the applicator 104 may be a handheld applicator such that the present invention provides an effective sterilisation apparatus which is easily transportable by a user. For example, the generator 102 may be powered by a battery or the like.

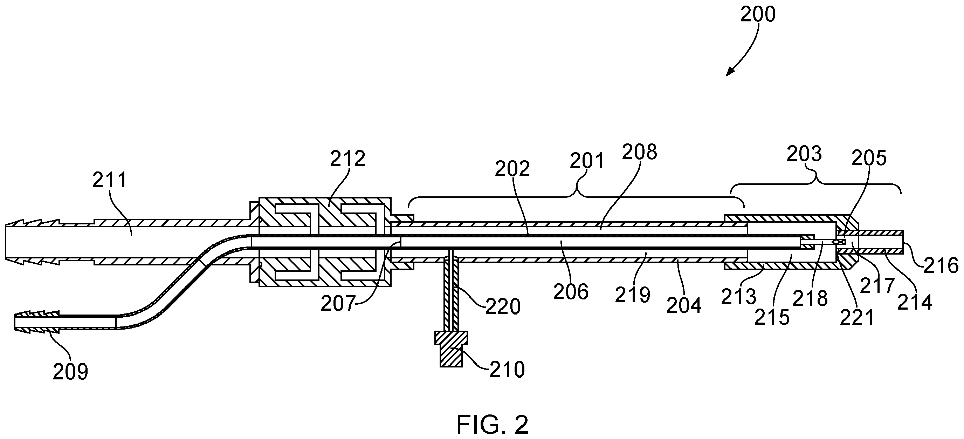

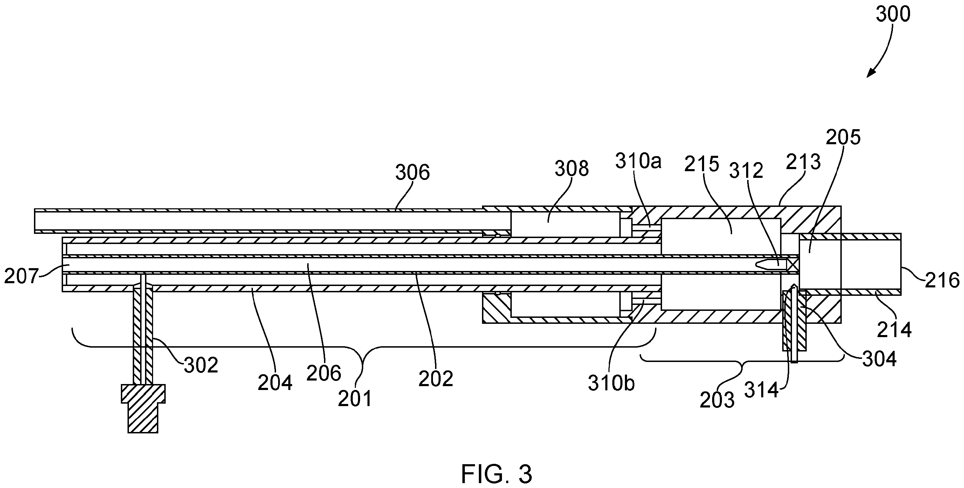

Examples of the applicator 104 are shown in more detail in Figs. 2 and 3 below. To sterilise a surface, a plasma is created in the applicator 104 by applying energy from the generator 102 to the gas delivered from the gas supply 110. For example, RF energy may be used to strike a plasma and microwave energy may be used to sustain the plasma. For example, plasma may be generated as disclosed in WO 2009/060213 A1. Simultaneous with the generation of plasma, water from the water supply 108 is passed to a hydroxyl radical generating region within the applicator 104 where the plasma ionises the water in order to produce a spray 112 of hydroxyl radicals which pass out of the applicator 104 to be directed at a surface or into an area for sterilisation. Examples of hydroxyl radical generation in this manner are disclosed in WO 2009/060214 A1, for example.

The applicator 104 may be produced at any suitable scale. For example, the applicator may be sized to be gripped by a human hand. Alternatively, a larger version suitable for mounted on a stand may be manufactured. In use, the stream of plasma and/or OH radicals emitted by the applicator may be directed into a volume to be sterilized, e.g. the inside of a vehicle (e.g. ambulance) or a hospital bed or surgical suite.

Fig. 2 shows a cross-sectional view of an applicator 200 that is a first embodiment of the invention. Although not shown in Fig. 2, the applicator 200 may be contained within a generally elongate housing which allows a user to easily pass the applicator 200 over a surface or object for sterilisation. In particularly preferred embodiments the applicator 200 may be handheld unit to facilitate manual control.

The applicator 200 comprises an energy delivery structure in the form of a coaxial transmission line 201 for conveying radiofrequency (RF) and/or microwave frequency electromagnetic (EM) energy. The coaxial transmission line 201 comprises an inner conductor 202 and an outer conductor 204 spaced away from the inner conductor 202 to define an annular region 219 therebetween. For example, in a preferred embodiment the inner conductor 202 may have an outer diameter of 3 mm and the outer conductor 204 may have an inner diameter of 7 mm to provide a suitable spacing. The spacing between the inner conductor 202 and the outer conductor 204 may be maintained by radially extending spacers (not shown) which are positioned in the gap, for example the spacers may be spokes or spoked discs made of PTFE.

A distal tip 203 is mounted at a distal end of the coaxial transmission line 201. The distal tip 203 comprises a cylindrical cap 213, which is an electrically conductive structure electrically connected to the outer conductor 204 of the coaxial transmission line 201. In this embodiment, the cylindrical cap 213 comprises a proximal region that overlies and contacts an outer surface of the outer conductor 204. The cylindrical cap 213 defines an internal volume 215. The inner conductor 202 of the coaxial transmission line 201 protrudes beyond a distal end of the outer conductor 204 into the internal volume. The cylindrical cap 215 has an outlet aperture 217 in its distal end. The internal volume 215 is in fluid communication with an external environment through the outlet aperture 217. In this example, an insulating tube 214 (e.g. formed from quartz or the like) is mounted in the outlet aperture, such that the internal volume 215 communicates with the external environment through a passageway formed by the insulating tube 214.

The inner conductor 202 is hollow to form a water conduit 206 for conveying water along the coaxial transmission line 201 from a proximal inlet 207 to the internal volume 215 within the distal tip 203. A stream of water is fed into the proximal inlet 207 via a water input pipe 209, such that the stream of water is parallel with the longitudinal axis of the inner conductor 202. This arrangements allows a high water flow rate due to the lack of curves or bends in the water conduit 206. The water input pipe 209 receives water from a pump or other water supply, as described above with respect to Fig. 1.

The annular region 219 between the inner conductor 202 and the outer conductor 204 forms a fluid conduit 208 for conveying gas to the internal volume 215. Gas is delivered to the fluid conduit 208 through a gas input pipe 211, which is connected to a gas supply as described above with respect to Fig. 1.

It is envisaged that the applicator 200 may also be operated by conveying a mixture of gas and a water mist through the fluid conduit 208. In such operation, no water is required to be delivered through the water conduit 206, thought water may be simultaneously supplied through the water conduit 206 if necessary.

RF and/or microwave energy is supplied to the coaxial transmission line 201 via a transverse coaxial feed 220. The transverse coaxial feed 220 couples the RF and/or microwave energy into the coaxial transmission line 201 at a location positioned towards a proximal end of the coaxial transmission line 201. To enable RF energy to be conveyed by the coaxial transmission line 201, the coaxial transmission line 201 has an open circuit condition at its proximal end (i.e. the inner conductor 202 and outer conductor 204 remain isolated from each other). To ensure efficient coupling of the microwave energy into this coaxial transmission line 201, the transverse coaxial feed is preferably positioned away from the proximal end of the coaxial transmission line by a distance equal to one or more half wavelengths of the microwave energy when propagating on the coaxial transmission line 201.

The transverse coaxial feed 220 has a connector 210 that is detachably connectable to a coaxial cable that conveys RF and/or microwave energy from a generator, as described above with respect to Fig. 1. For example, the connector 210 may comprise a QMA, a SMA, a N connector or the like.

In order to prevent microwave energy from flowing past the proximal end of the coaxial transmission line 201, a choke 212 is connected at the proximal end of the coaxial transmission line 201. In this example a double choke arrangement is used. The choke 212 is provided with a longitudinal passage therethrough to admit the water input pipe 209 and to provide fluid communication between the gas input pipe 211 and annular region 219.

As described above, the cylindrical cap 213 is open at its distal end, with an insulating tube 214 positioned within the outlet aperture 217. A proximal region of the insulating tube 214 defines a plasma generating zone 205. A first electrode 218 that is electrically connected to the inner conductor 202 extends into the plasma generating zone 205. In this example, the first electrode 218 is a hollow conductive rod that protrudes from a distal end of the inner conductor 202. The rod has a smaller outer diameter than the outer diameter of the inner conductor 202. The water conduit 206 may be in fluid communication with a longitudinal passage through the first electrode 218. The longitudinal passage may have a smaller diameter than that water conduit 206 so that the speed of water flow in the longitudinal passage is increased relative to the water conduit 206, i.e. the water accelerates towards the plasma generating region 205.

A spray nozzle is mounted at a distal end of the longitudinal passage. The spray nozzle may comprise a swirl chamber arranged to impart a vortex motion on the flow of water as it exits the longitudinal passage, so that a cone of water droplets or water mist is introduced to the plasma generating region 205.

A second electrode 221 is provided by one more radially protruding conductive tabs formed on the side surfaces of the outlet aperture 217 at a proximal end of the insulating tube 214. Energy supplied to the coaxial transmission line 201 may thus cause a high voltage condition to exist between the first electrode 218 and second electrode 221 within the plasma generating zone 205, such that a plasma can be struck from gas supplied through the fluid conduit 208. The plasma may be struck by a pulse of RF energy and then sustained by a subsequent microwave EM pulse or pulses. In other embodiments either RF or microwave EM energy alone may be used to strike and/or to sustain the plasma.

A benefit of forming the second electrode as discrete tabs is that it has less effect on the impedance in the cylindrical cap, and hence assists in efficient coupling of energy through the apparatus.

The conductive tabs may be arranged evenly around the outlet aperture 217. For example there may be two opposed conductive tabs, or four conductive tabs arranged at 90° intervals around the outlet aperture. The conductive tabs provide locations in which arcing preferentially occurs between conductive elements connected to the inner conductor and outer conductor of the coaxial transmission line. That is, arcing and hence plasma generation, occurs preferentially between the first electrode 218 and the second electrode 221. The relative dimensions of the first electrode 218 and second electrode 221 are selected in conjunction with the power supplied to the plasma generating region in order to achieve an electric field strength to strike and sustain the plasma. Where the gas is argon, the field strength required for breakdown may be 600 $Vmm^{-1}$, for example. For example, the first electrode 218 may have an outer diameter of 0.5 mm, and the second electrodes 221 may be radially spaced from the first electrode 218 by a distance equal to or less than 1 mm.

The insulating tube 214 covers the side surface of the outlet aperture 217 beyond the plasma generating zone 205 to avoid unwanted arcing in locations away from the first and second electrode.

The plasma may be naturally directed out of a distal end of the insulating tube 214 by the direction of the gas flow from the gas input pipe 211.

Meanwhile, the hollow inner conductor 202 conveys water or mist via the water conduit 206 to the longitudinal passage in the first electrode 218 and onwards as a spray into the plasma generating zone 205. Here the plasma ionises the water molecules to product hydroxyl radicals, which then flow out of the applicator 200. The insulating tube 214 may have an inner diameter selected to narrow the outlet aperture 217 in a manner increases the speed of gas as it exits the applicator. This may aid dispersal of hydroxyl radicals over a region to be sterilised. For example, the insulating tube 214 may have an outer diameter of 10 mm and an inner diameter of 8 mm.

As explained above, in one example, the first electrode 218 is itself a hollow tube that forms a distal portion of the water conduit 206. The first electrode may have at its distal tip an aerosoliser, i.e. a spray head configured to generate fine droplets from a stream of water provided through the water conduit 206. For example, the aerosoliser may be configured to generate a conical spray of water mist to be directed into the plasma generating zone 205.

However, in another embodiment, the applicator 200 may be operated by delivering a mixture of a gas and a water mist through the inlet 211 and through the fluid conduit 208. The inner conductor 202 and first electrode 218 need not be hollow in this arrangement. When operating in this way, a plasma may be generated at the plasma generating zone 205 to ionise water molecules and provide hydroxyl radicals in substantially the same manner as described above.

Fig. 3 shows an applicator 300 which is another embodiment of the invention. Features of the applicator 300 which correspond with the applicator 200 discussed above with respect to Fig. 2 are given the same reference numerals, and are not described again.

In the applicator 300, energy is coupled into the applicator 300 using two feeds 302, 304 that are mounted transverse to the longitudinal axis of the coaxial transmission line 201. A first feed 302 is connected towards a proximal end of the coaxial transmission line 201. The first feed 302 is a coaxial feedline configured to couple microwave frequency EM energy into the coaxial transmission line 201. In this example, a proximal end of the coaxial transmission line 201 is in a short circuit condition (i.e. the inner conductor 202 is electrically connected to the outer conductor 204). The first feed 302 is then positioned at one or an odd multiple quarter-wavelength distance (at the microwave frequency) from the proximal end of the coaxial transmission line 201. For example, for a microwave frequency of 5.8 GHz, the first feed 302 may be positioned a distance of around 13 mm from the proximal end of the coaxial transmission line 201.

A second feed 304 is provided at a distal end of the applicator 300, through a side wall of the cylindrical cap 213 into the plasma generating zone 205. The second feed 304 is a coaxial feedline configured to couple RF EM energy into the plasma generating zone 205. To avoid the second feed 304 coupling out microwave energy, it is desirably placed at one or more half wavelengths from the short circuit condition at the proximal end of the coaxial transmission line 201.

The second feed 304 may be configured as an igniter for delivering a RF pulse having a voltage capable of striking a thermal or non-thermal plasma in the plasma generating zone 205. The second feed 304 comprises a strike electrode 314 which protrudes into the plasma generating zone 205 to ensure that plasma is struck at the correct place.

The first feed 302 and the second feed 304 may receive microwave and RF energy respectively from different sources, or via separate feeds from a generator configured to produce both RF and microwave signals.

In this example, the applicator 300 comprises a gas duct 306 which is parallel to the coaxial transmission line 201. Gas, such as argon, is fed into the gas duct from a gas supply as described above with reference to Fig. 1. At a distal end, the gas duct 306 directs gas into a chamber 308 which encircles the coaxial transmission line 201 at a proximal end of the cylindrical cap 203. Gas flows from the chamber 308 into the internal volume 215 of the cylindrical cap 203 through a number of openings 310a, 310b formed in a proximal end surface of the cylindrical cap 203. The openings 310a, 310b are radially spaced around the coaxial transmission line 201 to ensure even distribution of gas in the internal volume 215.

As in the arrangement shown in Fig. 2, the inner conductor 202 of the coaxial transmission line 201 is hollow to provide a water conduit 206. The inner conductor 202 protrudes beyond a distal end of the outer conductor 204 into the internal volume 215. A distal portion of the inner conductor 202 and a surrounding annular conductive provided by the cylindrical cap 213 provide a first electrode and second electrode respectively for coupling microwave energy from the coaxial transmission line 201 into plasma formed in the plasma generating zone 205. Plasma may thus be struck using an RF pulse from the second feed 304 and sustained by microwave energy from the first feed 302.

An aerosoliser 312 is disposed within a distal end of the inner conductor 202. The aerosoliser 312 is configured to produce a conical spray of water mist in the plasma generating zone 205. To produce hydroxyl radicals for sterilisation, water is passed through the water conduit 206 to generate a water mist directed outwards from the aerosoliser 312. At the same time, gas is passed into the fluid conduit 208 from the gas duct 306 to ensure a stream of gas also passes through the plasma generating zone 205. An RF pulse is delivered through the second feed 304 in order to strike a thermal or non-thermal plasma from the gas. The plasma is sustained using a pulse or pulses of microwave EM energy supplied to the coaxial transmission line 201 from the first feed 302. The plasma which is generated in this way ionises water molecules in the water mist, to produce a spray of hydroxyl radicals directed out of the plasma generating zone 205 and through the outlet 216 towards a region to be sterilised.

**Claims**

1. A sterilisation device (200, 300) for generating a flow of hydroxyl radicals, the sterilisation device comprising:

   a coaxial transmission line (201) for conveying radiofrequency (RF) and/or microwave frequency electromagnetic (EM) energy, the coaxial transmission line (201) extending in a longitudinal direction and comprising an inner conductor (202) and an outer conductor (204) located around and spaced away from the inner conductor (202);

an end cap (213) mounted on a distal end of the coaxial transmission line (201), wherein the end cap (213) comprises a distally facing outlet aperture (217);

a fluid conduit (208) extending in the longitudinal direction from a fluid inlet at a distal end of the coaxial transmission line through the end cap (213) to the outlet aperture (217); and

a plasma generating region (205) at a proximal end of the outlet aperture (217), wherein the plasma generating region (205) contains a first electrode (218) that is electrically connected to the inner conductor (202), and a second electrode (221) that is electrically connected to the outer conductor (204),

wherein the fluid conduit (206, 306) defines a longitudinal fluid flow path through the device (200, 300) that is aligned with a feed direction in which fluid is receivable through the fluid inlet, and

wherein the first electrode (218) and second electrode (221) oppose each other in a transverse direction across the longitudinal fluid flow path in the plasma generating region (205).

2. The sterilisation device (200) of claim 1, wherein the fluid conduit includes a passage (208) between the inner conductor (202 and outer conductor (204) of the coaxial transmission line; or wherein the fluid conduit includes a duct that runs parallel to the coaxial transmission line.

3. The sterilisation device (200, 300) of claim 1 or claim 2 further comprising a water conduit (206) arranged to deliver water to the plasma generating region (205).

4. The sterilisation device (200, 300) of claim 3, wherein the water conduit (206) includes a longitudinal passageway formed within the inner conductor (202) of the coaxial transmission line (201), and wherein the water conduit includes a longitudinal passageway formed within the first electrode.

5. The sterilisation device (200, 300) of claim 3 or claim 4 further comprising a spray unit at a distal end of the water conduit (206), wherein the spray unit comprises an aerosoliser (312) configured to produce a cone-shaped spray of water mist.

6. The sterilisation device (200, 300) of any one of claims 3 to 5, wherein the water conduit (206) has a proximal inlet, and wherein the water conduit (206) defines a longitudinal flow path through the device (200, 300) that is aligned with a feed direction in which water is receivable through the proximal inlet.

7. The sterilisation device (200, 300) of any preceding claim, wherein the first electrode (208) is a rod that protrudes in the longitudinal direction from a distal end of the inner conductor (202), the rod having a smaller diameter than the inner conductor (202).

8. The sterilisation device (200, 300) of any preceding claim, wherein the plasma generating region (205) is located in a proximal region of the outlet aperture (217), and wherein the second electrode (221) comprises a plurality of radial tabs extending inwards from a side wall of the outlet aperture (217) .

9. The sterilisation device (200, 300) of any preceding claim further comprising an insulating tube (214) mounted in the outlet aperture (217) distally from the plasma generating region (205).

10. The sterilisation device (200, 300) of any preceding claim further comprising a transverse coaxial feed (220, 302) connected to introduce the RF and/or microwave energy to the coaxial transmission line (201) in a proximal region thereof, wherein the transverse coaxial feed (220, 302) is configured to couple microwave energy into the coaxial transmission line (201), and wherein the transverse coaxial feed (220, 302) is connected to a point on the coaxial transmission line (201) located at a distance $\frac{(2n-1)\lambda}{4}$ from a proximal end thereof, where n is a positive integer, and $\lambda$ is a wavelength of the microwave energy conveyed by the coaxial transmission line (201).

11. The sterilisation device (200, 300) of claim 10 further comprising a proximal transverse coaxial feed (304) connected to introduce the RF energy directly to the plasma generating region (205).

12. A sterilisation apparatus (100) comprising:

   the sterilisation device (200, 300) of any preceding claim;
   a water supply (108) connected to supply water to the plasma generating region (205);
   a gas supply (110) connected to supply gas to the plasma generating region (205) via the fluid conduit (208); and
   a generator (102) connected to supply radiofrequency (RF) and/or microwave frequency electromagnetic energy to the plasma generating region (205).

13. The sterilisation apparatus (100) of claim 12, wherein the water supply (108) comprises a pump; or wherein the water supply comprises a mist generator.

14. The sterilisation apparatus (100) of claim 13, wherein the mist generator comprises either:

   an ultrasonic transducer, or
   a heating element.

15. The sterilisation apparatus (100) of claim 13 or 14, wherein the gas supply (110) is connected to supply gas to the sterilisation device (200, 300) via the mist generator.

**Patentansprüche**

1. Sterilisationsvorrichtung (200, 300) zur Erzeugung eines Stroms von Hydroxyl-Radikalen, wobei die Sterilisationsvorrichtung Folgendes umfasst:

   eine koaxiale Übertragungsleitung (201) zur Beförderung von Hochfrequenz- (HF-) und/oder elektromagnetischer Mikrowellen- (EM-) Energie, wobei sich die koaxiale Übertragungsleitung (201) in eine Längsrichtung erstreckt und einen inneren Leiter (202) und einen äußeren Leiter (204), der sich um den inneren Leiter (202) herum und von diesem beabstandet befindet, umfasst;
   eine Endkappe (213), die am distalen Ende der koaxialen Übertragungsleitung (201) angebracht ist, wobei die Endkappe (213) eine distal ausgerichtete Auslassöffnung (217) umfasst;
   eine Fluidleitung (208), die sich in Längsrichtung von einem Fluideinlass am distalen Ende der koaxialen Übertragungsleitung durch die Endkappe (213) zur Auslassöffnung (217) erstreckt; und
   einen Plasmaerzeugungsbereich (205) an einem proximalen Ende der Auslassöffnung (217), wobei der Plasmaerzeugungsbereich (205) eine erste Elektrode (218), die elektrisch mit dem inneren Leiter (202) verbunden ist, und eine zweite Elektrode (221), die elektrisch mit dem äußeren Leiter (204) verbunden ist, umfasst,
   wobei die Fluidleitung (206, 306) einen Längs-Fluidströmungspfad durch die Vorrichtung (200, 300) definiert, der mit einer Einspeisungsrichtung fluchtend ausgerichtet ist, in der Fluid durch den Fluideinlass aufnehmbar ist, und
   wobei die erste Elektrode (218) und die zweite Elektrode (221) in einer Querrichtung quer zum Längs-Fluidströmungspfad im Plasmaerzeugungsbereich (205) einander gegenüberliegen.

**2.** Sterilisationsvorrichtung (200) nach Anspruch 1, wobei die Fluidleitung einen Durchgang (208) zwischen dem inneren Leiter (202) und dem äußeren Leiter (204) der koaxialen Übertragungsleitung umfasst, oder wobei die Fluidleitung einen Kanal umfasst, der parallel zur koaxialen Übertragungsleitung verläuft.

**3.** Sterilisationsvorrichtung (200, 300) nach Anspruch 1 oder Anspruch 2, die weiters eine Wasserleitung (206) umfasst, die angeordnet ist, um Wasser zum Plasmaerzeugungsbereich (205) zuzuführen.

**4.** Sterilisationsvorrichtung (200, 300) nach Anspruch 3, wobei die Wasserleitung (206) einen Längs-Durchgangspfad umfasst, der im inneren Leiter (202) der koaxialen Übertragungsleitung (201) ausgebildet ist, und wobei die Wasserleitung einen Längs-Durchgangspfad umfasst, der in der ersten Elektrode ausgebildet ist.

**5.** Sterilisationsvorrichtung (200, 300) nach Anspruch 3 oder Anspruch 4, die weiters eine Sprüheinheit am distalen Ende der Wasserleitung (206) umfasst, wobei die Sprüheinheit einen Zerstäuber (312) umfasst, der ausgelegt ist, um einen kegelförmigen Wassernebel zu erzeugen.

**6.** Sterilisationsvorrichtung (200, 300) nach einem der Ansprüche 3 bis 5, wobei die Wasserleitung (206) einen proximalen Einlass aufweist und wobei die Wasserleitung (206) einen Längs-Strömungspfad durch die Vorrichtung (200, 300) definiert, der mit einer Einspeisungsrichtung fluchtend ausgerichtet ist, in der Wasser durch den proximalen Einlass aufnehmbar ist.

**7.** Sterilisationsvorrichtung (200, 300) nach einem der vorangegangenen Ansprüche, wobei die erste Elektrode (208) ein Stab ist, der in Längsrichtung vom distalen Ende des inneren Leiters (202) vorsteht, wobei der Stab einen kleineren Durchmesser aufweist als der innere Leiter (202).

**8.** Sterilisationsvorrichtung (200, 300) nach einem der vorangegangenen Ansprüche, wobei sich der Plasmaerzeugungsbereich (205) in einem proximalen Bereich der Auslassöffnung (217) befindet und wobei die zweite Elektrode (221) eine Vielzahl von radialen Laschen umfasst, die sich von einer Seitenwand der Auslassöffnung (217) nach innen erstrecken.

**9.** Sterilisationsvorrichtung (200, 300) nach einem der vorangegangenen Ansprüche, die weiters einen Isolierschlauch (214) umfasst, der in der Auslassöffnung (217) distal zum Plasmaerzeugungsbereich (205) befestigt ist.

**10.** Sterilisationsvorrichtung (200, 300) nach einem der vorangegangenen Ansprüche, die weiters eine koaxiale Quereinspeisung (220, 302) umfasst, die verbunden ist, um die HF- und/oder Mikrowellenenergie zur koaxialen Übertragungsleitung (201) in einem proximalen Bereich davon einzubringen, wobei die koaxiale Quereinspeisung (220, 302) ausgelegt ist, um Mikrowellenenergie in die koaxiale Übertragungsleitung (201) einzukoppeln, und wobei die koaxiale Quereinspeisung (220, 302) mit einem Punkt auf der koaxialen Übertragungsleitung (201) verbunden ist, der sich in einem Abstand $\frac{(2n-1)\lambda}{4}$ von deren proximalen Ende befindet, worin n eine positive ganze Zahl ist und $\lambda$ die Wellenlänge der Mikrowellenenergie ist, die von der koaxialen Übertragungsleitung (201) befördert wird.

**11.** Sterilisationsvorrichtung (200, 300) nach Anspruch 10, die weiters eine proximale koaxiale Quereinspeisung (304) umfasst, die verbunden ist, um die HF-Energie direkt zum Plasmaerzeugungsbereich (205) einzubringen.

**12.** Sterilisationsgerät (100), das Folgendes umfasst:

eine Sterilisationsvorrichtung (200, 300) nach einem der vorangegangenen Ansprüche;
eine Wasserversorgung (108), die verbunden ist, um Wasser zum Plasmaerzeugungsbereich (205) zuzuführen;
eine Gasversorgung (110), die verbunden ist, um Gas zum Plasmaerzeugungsbereich (205) über die Fluidleitung (208) zuzuführen; und
einen Generator (102), der verbunden ist, um Hochfrequenz- (HF-) und/oder elektromagnetische Mikrowellenfrequenzenergie zum Plasmaerzeugungsbereich (205) zuzuführen.

**13.** Sterilisationsgerät (100) nach Anspruch 12, wobei die Wasserversorgung (108) eine Pumpe umfasst; oder wobei die Wasserversorgung einen Nebelgenerator umfasst.

**14.** Sterilisationsgerät (100) nach Anspruch 13, wobei der Nebelgenerator eines der Folgenden umfasst:

einen Ultraschallwandler oder
ein Heizelement.

**15.** Sterilisationsgerät (100) nach Anspruch 13 oder 14, wobei die Gasversorgung (110) verbunden ist, um Gas zur Sterilisationsvorrichtung (200, 300) über den Nebelgenerator zuzuführen.

**Revendications**

1. Dispositif de stérilisation (200, 300) pour générer un flux de radicaux hydroxyles, le dispositif de stérilisation comprenant :

   une ligne de transmission coaxiale (201) pour transporter de l'énergie électromagnétique (EM) radiofréquence (RF) et/ou hyperfréquence, la ligne de transmission coaxiale (201) s'étendant dans une direction longitudinale et comprenant un conducteur interne (202) et un conducteur externe (204) situé autour et à distance du conducteur interne (202) ;
   un capuchon d'extrémité (213) monté sur une extrémité distale de la ligne de transmission coaxiale (201), dans lequel le capuchon d'extrémité (213) comprend une ouverture de sortie orientée de manière distale (217) ;
   un conduit de fluide (208) s'étendant dans la direction longitudinale à partir d'une entrée de fluide au niveau d'une extrémité distale de la ligne de transmission coaxiale à travers le capuchon d'extrémité (213) vers l'ouverture de sortie (217) ; et
   une région de génération de plasma (205) au niveau d'une extrémité proximale de l'ouverture de sortie (217), dans lequel la région de génération de plasma (205) contient une première électrode (218) qui est connectée électriquement au conducteur interne (202), et une seconde électrode (221) qui est connectée électriquement au conducteur externe (204),
   dans lequel le conduit de fluide (206, 306) définit un trajet d'écoulement de fluide longitudinal à travers le dispositif (200, 300) qui est aligné avec une direction d'alimentation dans laquelle du fluide peut être reçu à travers l'entrée de fluide, et
   dans lequel la première électrode (218) et la seconde électrode (221) sont opposées l'une à l'autre dans une direction transversale à travers le trajet d'écoulement de fluide longitudinal dans la région de génération de plasma (205).

2. Dispositif de stérilisation (200) selon la revendication 1, dans lequel le conduit de fluide inclut un passage (208) entre le conducteur interne (202) et le conducteur externe (204) de la ligne de transmission coaxiale ; ou
   dans lequel le conduit de fluide inclut un conduit qui s'étend parallèlement à la ligne de transmission coaxiale.

3. Dispositif de stérilisation (200, 300) selon la revendication 1 ou la revendication 2, comprenant en outre un conduit d'eau (206) agencé pour délivrer de l'eau à la région de génération de plasma (205).

4. Dispositif de stérilisation (200, 300) selon la revendication 3, dans lequel le conduit d'eau (206) inclut un passage longitudinal formé à l'intérieur du conducteur interne (202) de la ligne de transmission coaxiale (201), et dans lequel le conduit d'eau inclut un passage longitudinal formé à l'intérieur de la première électrode.

5. Dispositif de stérilisation (200, 300) selon la revendication 3 ou la revendication 4, comprenant en outre une unité de pulvérisation au niveau d'une extrémité distale du conduit d'eau (206), dans lequel l'unité de pulvérisation comprend un aérosoliseur (312) configuré pour produire une pulvérisation en forme de cône de brouillard d'eau.

6. Dispositif de stérilisation (200, 300) selon l'une quelconque des revendications 3 à 5, dans lequel le conduit d'eau (206) présente une entrée proximale, et dans lequel le conduit d'eau (206) définit un trajet d'écoulement longitudinal à travers le dispositif (200, 300) qui est aligné avec une direction d'alimentation dans laquelle de l'eau peut être reçue à travers l'entrée proximale.

7. Dispositif de stérilisation (200, 300) selon l'une quelconque des revendications précédentes, dans lequel la première électrode (208) est une tige qui fait saillie dans la direction longitudinale à partir d'une extrémité distale du conducteur interne (202), la tige présentant un diamètre plus petit que le conducteur interne (202).

8. Dispositif de stérilisation (200, 300) selon l'une quelconque des revendications précédentes, dans lequel la région de génération de plasma (205) est située dans une région proximale de l'ouverture de sortie (217), et dans lequel la seconde électrode (221) comprend une pluralité de pattes radiales s'étendant vers l'intérieur à partir d'une paroi latérale de l'ouverture de sortie (217).

9. Dispositif de stérilisation (200, 300) selon l'une quelconque des revendications précédentes, comprenant en outre un tube isolant (214) monté dans l'ouverture de sortie (217) de manière distale par rapport à la région de génération de plasma (205).

10. Dispositif de stérilisation (200, 300) selon l'une quelconque des revendications précédentes, comprenant en outre une alimentation coaxiale transversale (220, 302) connectée pour introduire l'énergie RF et/ou hyperfréquence dans la ligne de transmission coaxiale (201) dans une région proximale de celle-ci, dans lequel l'alimentation coaxiale transversale (220, 302) est configurée pour coupler de l'énergie hyperfréquence dans la ligne de transmission coaxiale (201), et dans lequel l'alimentation coaxiale transversale (220, 302) est connectée à un point sur la ligne de transmission coaxiale (201) situé à une

distance $\frac{(2n-1)\lambda}{4}$ d'une extrémité proximale de celle-ci, où n est un nombre entier positif, et $\lambda$ est une longueur d'onde de l'énergie hyperfréquence transportée par la ligne de transmission coaxiale (201).

11. Dispositif de stérilisation (200, 300) selon la revendication 10, comprenant en outre une alimentation coaxiale transversale proximale (304) connectée pour introduire l'énergie RF directement dans la région de génération de plasma (205).

12. Appareil de stérilisation (100) comprenant : le dispositif de stérilisation (200, 300) selon l'une quelconque des revendications précédentes ;

   une alimentation en eau (108) connectée pour fournir de l'eau à la région de génération de plasma (205) ;
   une alimentation en gaz (110) connectée pour fournir du gaz à la région de génération de plasma (205) via le conduit de fluide (208) ; et
   un générateur (102) connecté pour fournir de l'énergie électromagnétique radiofréquence (RF) et/ou hyperfréquence à la région de génération de plasma (205).

13. Appareil de stérilisation (100) selon la revendication 12, dans lequel l'alimentation en eau (108) comprend une pompe ; ou dans lequel l'alimentation en eau comprend un générateur de brouillard.

14. Appareil de stérilisation (100) selon la revendication 13, dans lequel le générateur de brouillard comprend soit :

   un transducteur ultrasonore, soit
   un élément chauffant.

15. Appareil de stérilisation (100) selon la revendication 13 ou 14, dans lequel l'alimentation en gaz (110) est connectée pour fournir du gaz au dispositif de stérilisation (200, 300) via le générateur de brouillard.

FIG. 1

FIG. 2

EP 4 142 812 B1

FIG. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009060214 A
- WO 2019175063 A
- WO 2019175063 A1
- GB 2548382 A
- GB 2463521 A
- WO 2012076844 A
- WO 2009060213 A1
- WO 2009060214 A1

**Non-patent literature cited in the description**

- **BAI et al.** Experimental studies on elimination of microbial contamination by hydroxyl radicals produced by strong ionisation discharge. *Science and Technology,* August 2008, vol. 10 (4